# EUROPEAN PATENT SPECIFICATION

(11) **EP 3 648 805 B1**
(45) Date of publication and mention of the grant of the patent: **12.10.2022**
(21) Application number: 18752260.2
(22) Date of filing: 06.07.2018
(51) Int. Cl.: A61L 27/04, A61L 27/06, A61L 27/30, A61L 27/34

(54) **FUNCTIONALIZED-PIEZOELECTRIC-COATINGS-DRIVEN BIOMICROELECTROMECHANICAL SYSTEMS (BIOMEMS) FOR ENHANCED METALLIC IMPLANTS PERFORMERS**
ÜBER FUNKTIONALISIERTE PIEZOELEKTRISCHE BESCHICHTUNGEN ANGESTEUERTE BIOMIKROELEKTROMECHANISCHE SYSTEME (BIOMEMS) FÜR VERBESSERTE LEISTUNG VON METALLISCHEN IMPLANTATEN
BIOMICROSYSTÈMES ÉLECTROMÉCANIQUES (BIOMEMS) ENTRAÎNÉS PAR DES REVÊTEMENTS PIÉZOÉLECTRIQUES FONCTIONNALISÉS POUR IMPLANTS MÉTALLIQUES AMÉLIORÉS

(30) Priority: 07.07.2017 PT 2017110191
(43) Date of publication of application: 13.05.2020
(73) Proprietor: Universidade De Aveiro, 3810-193 Aveiro (PT)
(72) Inventor: LOUSADA SILVEIRINHA VILARINHO, Paula Maria, 3810-046 Aveiro (PT); CZESLAW ZLOTNIK, Sebastian, 3810-104 Aveiro (PT); VIANA MALTEZ DA COSTA, Marisa Maria, 3810-120 Aveiro (PT); FERNANDES, Marisa Helena, 3800-110 Aveiro (PT)
(74) Representative: Ferreira, Maria Silvina
(86) International application number: PCT/IB2018/055015
(87) International publication number: WO 2019/008552

(56) References cited:
- WO-A1-95/19796
- US-A1- 2012 253 104
- BARROCA NATHALIE ET AL: "Stability of electrically induced-polarization in poly (L-lactic) acid for bone regeneration", APPLIED PHYSICS LETTERS, A I P PUBLISHING LLC, US, vol. 101, no. 2, 9 July 2012 (2012-07-09), pages 23701-23701, XP012163903, ISSN: 0003-6951, DOI: 10.1063/1.4729619 [retrieved on 2012-07-10]
- VILARINHO PAULA MARIA ET AL: "Are lithium niobate (LiNbO3) and lithium tantalate (LiTaO3) ferroelectrics bioactive?", MATERIALS SCIENCE AND ENGINEERING C, vol. 39, 2014, pages 395-402, XP029029228, ISSN: 0928-4931, DOI: 10.1016/J.MSEC.2014.03.026
- SATAPATHY S ET AL: "Structural, dielectric and ferroelectric properties of multilayer lithium tantalate thin films prepared by sol gel technique", THIN SOLID FILMS, ELSEVIER, AMSTERDAM, NL, vol. 519, no. 6, 4 October 2010 (2010-10-04), pages 1803-1808, XP028171324, ISSN: 0040-6090, DOI: 10.1016/J.TSF.2010.10.016 [retrieved on 2010-10-19]

## Description

### Technical Domain

The present application relates to a Bio MicroElectroMechanical System device (BioMEMs) and a method to produce said BioMEMs.

### Background Art

The design of new biomaterials for hard tissue repair is highly required, and bone tissue engineering has been viewed as an important field to support regenerative medicine. Bone is a quite diverse tissue with extraordinary properties, structure and functions, providing protection to the main organs [1], serving important metabolic activities and assisting locomotion of the body. Its repair, replacement or regeneration is crucial in clinical treatments. The osseous tissue is known to have self-healing and remodelling ability, though large-scale bone defects cannot be completely healed, leading to the need of an external intervention [2]. The development of artificial implant materials is generally based on mimicking the structure and function of the biological systems, in this case the bone.

A wide range of synthetic materials, metals, polymers and ceramics have been used as bone substitutes [3]. Among ceramics, calcium phosphate-based materials are some of the most attractive owing to their similarities to bone composition. Even though, due to their superior mechanical strength and chemical/biological inertness metals and their alloys are important structural biomaterials in orthopaedics [4]. Among the approved metals and alloys routinely used in clinical practice, one of the most common is austenitic stainless steel being applied as temporary devices (fracture plates, screws, hip nails) and long-term prosthesis (total hip replacements) [4]. Type 316L stainless steel (U.S. Food and Drug Administration (FDA) approved) and its variants are quite popular in implant technology because of their availability, low cost, very good workability and ability to be processed and shaped by several techniques, relatively good biocompatibility, as well as good toughness, high strength and controllable mechanical properties [4]. However, its poor bioactivity leads to problematic unsuccessful osseointegration.

Indeed, currently implant strategies face numerous difficulties and some adverse effects arising from poor biological interaction at the implant interface that can result in failures and related infection, highly painful for the patient and expensive for the society [5]. For instance, infections associated to orthopaedic fracture and reconstructive devices occur in up to 10 % of cases, mainly due to mechanical aseptic loosening of the implant [6-7].

Previously, orthopaedic implants were designed simply as mechanical functional devices. However, the above described limitations are requiring different approaches. With interest might be the creation of functional bioactive implant surfaces by coating the implants with a functionalized material to improve acceptance from the host tissue [6]. Besides exploring the numerous possibilities of local drug delivery to modulate *in situ* tissue reactions, it is of interest if the implant coating itself would facilitate more robust osseointegration at earlier stages [6].

Biomedical metals in particular due to their excellent mechanical properties, are commonly used in clinical treatments as supporting or fixation elements. Recently and leveraged by the growing needs of medical care these biomedical metals should provide more functions in addition to the mechanical ones, such as bioactivation, bone tissue inducing, anti-microbial, anti-tumor, anticoagulation, and drug loading functions. This implies a major transformation of the biomedical metal and surface modification is still the most effective way to bio-functionalize the traditional base material, which endows biomedical metals with different bio-functions [8].

Several types of surface modification of metallic implants are reported in the literature as promising strategies to improve tissue tolerance, osseointegration and implant corrosion resistance. Among the different approaches relevance is given to surface modification of the metal substrate with for example biocompatible coatings, as synthetic calcium phosphates (calcium phosphate (CaP), calcium apatites (CaAp) and/or calcium carbonate (CaCO₃) ) [9]. Hydroxyapatite (HAp, Ca₁₀ (PO₄)₆(OH)₂) is the major component of bone and synthetic HAp or fluorine hydroxyapatite (FHA) have been used as bone repair materials enhancing new tissue growth, and promoting healing. Metal-based functional bioactive ceramic composites combine the advantages of metals and ceramics, ie the good mechanical properties of metals and the functional bioactivity of the ceramic, but some long-term problems have been identified in layered-coated materials related with coating peeling and bacteria proliferation [9].

Another example refers to bioactive and biocompatible organic-inorganic hybrid coatings prepared by sol-gel method. They consist of an inorganic TiO₂ matrix in which different percentages of poly(epsilon-caprolactone) (PCL), a biodegradable and biocompatible polymer, were incorporated. The coatings were used to modify the surface of Ti-6AI4V substrates in order to improve their wear and corrosion resistance [10].

A more recent surface engineering modification of bare metallic substrates for improved cellular response and biocompatibility is with graphene oxide (GO). Owing to its outstanding chemical and mechanical properties, ease of surface modification and processing, and surface nano-architecture, GO-coated surfaces promote cell adhesion and growth, making it suitable for tissue engineering applications. GO-coated 316L SS surfaces showed increased hydrophilicity and biocompatibility with SHSY-5Y neuronal cells, which proliferated well and showed decreased reactive oxygen species expression [11].

In spite of these examples there is still the need for much more developments and improvements. For example, biomedical metals coated with HAp layer may become interesting bioactive artificial hip joints, but the adhesive strength between HAp layer and the substrate remains a problem, and this problem may cause the loosing of the implant. In addition, the acid alkali treatment and alkali heat treatment strongly modify the surface, and affects the cell proliferation [8].

On the other hand, it is known that *in vivo* endogenous electric potentials can control cell functions such as growth, migration [12-13] and mitosis rate, among others [14]. Additionally, ex *vivo* experiments testified that under electrical stimulation synchronous contractions of cultured cardiac constructs were induced [15], cell proliferation and expression of bone morphogenetic proteins in osteoblastic cell lines increased [16], and directed cell migration in epithelial tissues during wound healing [17], took place. It is worthwhile to mention that clinical electrical stimulation of living tissues demonstrated that growth, maintenance or regeneration of bones, tendons and ligaments were boosted. To complement it is given that bone under mechanical loading due to the piezoelectric nature of collagen and by the movement of ionic fluids within the structure generates electrical potentials [18]; this strongly assists bone remodelling [18-20]. Accordingly, electrically active materials / surfaces may have a potential attractive application as hard tissue implants [18].

Among these electrically active materials one can consider the class of electrets and the class of piezoelectrics. Electrets are dielectric materials that have a quasi-permanent electric charge or dipole polarization, based on their internal space charges. Electret behaviour is usually associated with polymeric materials in which space charges originate electric fields. The uniqueness of piezoelectrics in creating electrical potentials under stress (and vice versa) is an advantage over other non-piezoelectric materials, since there will be no need for an external electric field source. It was proven that on negatively charged surfaces of electrically poled barium titanate (BaTiO₃), induced a preferential formation of calcium phosphate and cell growth and proliferation may be tailored by the pulsed electrical stimulus [18], [21]. Although not yet certain there is a clear evidence that polarized surfaces are able to attract proteins and ions.

Due to their superior electromechanical and ferroelectric properties perovskite oxides are the major group of piezoelectric materials. When compared with polar polymers the piezoelectric coefficients of inorganic piezoelectrics are orders of magnitude higher. Within piezoelectrics lithium niobate (LiNbO₃, LNO) and lithium tantalate (LiTaO₃, LTO), have already been proposed as potential biomaterials and their biological responses examined [20, 22-25]. *In vitro* studies revealed enhanced proliferation rates and osteoblast function through mineral formation on positively and negatively charged surfaces of LNO [20]. On this ground these materials may be possible platforms for cell attachment and subsequently tissue growth templates, relevant for further integration with the host bone tissue. In addition, the recent studies of the present applicants on LNO and LTO powders classify them as bioactive ferroelectrics [26].

The present application discloses an alternative strategy, coating a biomedical metallic substrate with a functionalized ferroelectric / piezoelectric biocompatible oxide layer or with a functionalized piezoelectric biocompatible and biodegradable polymeric layer; i.e. the combination of a functional ferroelectric / piezoelectric layer with a metallic implant in which the piezoelectric might be a potential approach for successful osseointegration.

### Related Patents

Document CN 1785439 A discloses a coated active biologic piezoelectric layer on Ti substrate prepared from fluorohydroxy apatite (FHA) and barium titanate (BT) through cleaning the surface of Ti substrate, microarc oxidizing to form a porous oxide film layer, and electrophoretic deposition to form a FHABT layer on the surface of Ti substrate. Its advantages are easy control of F content, high implantation stability, binding power to interface, biocompatibility and bioactivity, and high effect to promote generation of biologic bone and taking part of energy conversion in human body. The present application differs from this one: i) the bioactive material is different: LTO versus Hap; ii) the substrate is different: stainless steel versus Ti; dense films versus porous films; sol-gel based deposition versus electrophoretic deposition; iii) the present application claims the use of a piezoelectric / ferroelectric material, not claimed in this patent; iv) the present application claims the functionalization of the piezoelectric coating, not claimed in this patent.

Document US2007/0122638 relates to a method for the production of a metallic substrate having a biocompatible surface and to the substrate that is produced by means of said method. The method comprises treatment of a metal, i.e., Ti, Ti alloys with Al, V, Ta, Nb, Ni, Fe, Mo or mixtures thereof, Ta, Ta alloys with Fe, Al, Cr, stainless steel, with a melt of calcium nitrate and an additional component which is an oxygen salt of Na, K, Li, Mg and mixtures thereof, said treatment being effected at 180-480 DEG C. for 0.1 to 12 hours. A substrate is obtained, wherein the overall layer thickness ranges from 10 to below 1600 nm and the fatigue strength of the substrate is in the same fatigue strength range as that of an untreated substrate at equal number of vibrations N. The present application differs from this one: i) the coating is different: LTO versus calcium based oxides; ii) the coating process is different; iii) the present application claims the use of a piezoelectric / ferroelectric material, not claimed in this patent; iv) the present application claims the functionalization of the piezoelectric coating, not claimed in this patent.

Document WO1999018892 discloses the use of the electrical properties of biopolymers, in particular, alpha -helical polypeptides and the homo- and co-polymers formed from alpha -hydroxy acids, in materials for tissue growth and repair and other biological applications is disclosed. The invention features a biodegradable device for tissue growth and/or repair having at least one tissue contacting surface and comprising an electrically charged synthetic biodegradable, biopolymeric, bioelectret material characterized by a bulk monopolar charge that produces an external electrostatic field. Electrets are formed in the material of the device upon the application of an electrical voltage. The resultant biodegradable, bioelectret biopolymeric device is useful for promoting tissue growth (e.g., growth of nerve or bone tissue) and also as an aid in the final integration of a biodegradable device following transplantation. The present application differs from this one: i) the coating is different: LTO versus polymers and PLLA versus alpha-helical polypeptides and the homo- and co-polymers formed from alpha-hydroxy acids; ii) the present application claims a coating process in this patent not clear what type of materials form is used (if coatings, scaffolds, dense bulk, fibbers, etc); iii) the present application claims the use of a piezoelectric / ferroelectric material, not an electret effect as claimed in this patent; these are two very different effects; iv) the present application claims the functionalization of the piezoelectric coating (via Corona discharge or UV light), not claimed in this patent.

Document US5759205 relates to a biocompatible implant having improved host tissue ingrowth capability and enhanced blood compatibility and comprises at least one tissue-contacting surface of an electrically charged material. The electrically charged material can be further chemically modified with covalently bonded activator molecules, which further promote host tissue ingrowth and adhesion to the implant and/or enhance blood compatibility.

The application comprises a biocompatible implant of electrically charged fluoropolymers with surface-coupled attachment factors. The tissue-contacting surfaces of the implant consist essentially of an electrically charged material which has been chemically modified with covalently bonded activator molecules which further promote host tissue ingrowth and adhesion to the implant; i.e. a fluoropolymer, and providing an electrical charge to the fluoropolymer. The fluoropolymer coating can be deposited onto the tissue-contacting surfaces of the implant by plasma spray, plasma polymerization, or other deposition techniques. Both dense and porous coatings can be deposited. The fluoropolymer coating can then be electrically charged using corona charge injection techniques known in the art. The present application differs from this one: i) the coating is different: LTO versus fluoropolymer; ii) the present application claims the use of a piezoelectric / ferroelectric material, not an electrect as claimed in this patent; these are two very different effects; iii) the present invention does not attach any activator molecules to the polymer surface versus this one in which an electrically charged material is chemically modified with covalently bonded activator molecules; iv) no mention to the used substrate; v) the present application claims the functionalization of the piezoelectric coating by UV light and corona poling versus only the use of corona charge injection technique to electrically charge the fluoropolymer (note that Corona Charging is well known and used industrial process to charge the surface of the polymers) . Vilarinho, P.M. et al.,

Materials Science and Engineering C 39 (2014), 395-402, discloses the formation of apatite-like structures on the surface of LiNbO₃ and LiTaO₃ powders after immersion in simulated body fluid (SBF).

### Summary

The present application refers to BioMicroElectroMechanical System (BioMEMs) targeted to promote bone regeneration comprising a 316L-type stainless steel substrate coated with ferroelectric / piezoelectric LiTaO₃ layers or with biocompatible / biodegradable piezoelectric polymer layers, wherein the layers are electrically functionalized and present high surface wettability and energy, high rate of calcium phosphate formation, high adsorption of proteins and no inflammatory reactions in vivo.

In the BioMicroElectroMechanical System the biomedical metallic substrate is 316L-type stainless steel.

In one embodiment in the BioMicroElectroMechanical System the biocompatible / biodegradable polymer layers are poly L-lactic acid (PLLA).

In one embodiment the present application refers to a method for obtaining a BioMicroElectroMechanical System (BioMEMs) comprising the preparation of ferroelectric / piezoelectric LTO precursor solutions, or biocompatible / biodegradable piezoelectric polymer precursor solutions, the deposition of ferroelectric / piezoelectric LTO layers / coatings or biocompatible / biodegradable piezoelectric polymer layers / coatings onto 316-type stainless steel substrates by spin-coating / dip-coating / spraying, repeating the said deposition cycle until the final coating thickness, crystallizing the said coatings at a temperature commensurate with the crystallization temperature of the deposited layers, and the functionalization of ferroelectric / piezoelectric LTO layers / coatings or biocompatible / biodegradable piezoelectric polymer layers / coatings via electric field or photofunctionalization via UV-irradiation, wherein the coatings functionalization with an electric field is done by corona poling conducted in the vicinity of an electrode with small radius of curvature, the corona tip, being located at a distance from the counter electrode which a grounded conductive plate, defined by the equipment, that acts as support for the coatings on the 316L-type stainless steel substrate; and wherein the coatings photofunctionalization occurs with a wavelength of the UV-light lower than 265 nm.

In another embodiment the formation of a LTO precursor solution comprises the preparation of an air-stable precursor solution by sol-gel method from environmentally friendly components, the metal precursors being mixed according to the required stoichiometric molar ratio of the metals, and the final precursor to be adjusted to a certain concentration.

In yet another embodiment the preparation of a precursor polymer solution is made by dissolving poly L-lactic acid pellets in 1,4 dioxane at between 70°C and 90°C, which is then stabilized at a temperature between 20°C and 30°C.

In another embodiment the deposition of the coatings comprises using the previously prepared precursor solutions, multiple deposition cycles of the precursor solutions until the final coating thickness is achieved, in between cycles, each of the coatings is dried between 200 and 400°C for 30 s to 10 min and crystallized between 550 and 650 °C for 1 to 5 min in static O₂ atmosphere by Rapid Thermal Annealing (RTA) .

The coatings functionalization with an electric field is by corona poling.

The coatings functionalization with an electric field by corona poling is conducted in the vicinity of an electrode with small radius of curvature, the corona tip, being located at a distance from the counter electrode, which is a grounded conductive plate, defined by the equipment, that acts as support for the coatings on the biomedical 316L-type stainless steel substrate.

In another embodiment a negative high dc voltage of up to 15 kV was applied for up to 60 min at up to 100 °C and kept constant while cooling down between 15°C and 25°C, more specifically to 20°C.

The wavelength of the UV-light is lower than 265 nm (UVC range).

This technology relates to a method in which biomedical metallic substrates coated with functionalized ferroelectric / piezoelectric coatings present a higher surface wettability and energy, a higher rate of calcium phosphate formation and a higher adsorption of BSA proteins and no inflammatory reactions for *in vivo* implanted functionalized ferroelectric / piezoelectric coated 316L-SST substrates. The technology now disclosed is useful for the fabrication of Bio MicroElectroMechanical System devices (BioMEMs) targeted to:
i) promotion of bone regeneration
ii) reduction of bone implant failure;
iii) substitution of permanent bone implants;
iv) biological *in vivo* or ex *vivo* tissue growth;
v) improvement of protein adsorption.

### General description

This application discloses BioMicroElectroMechanical Systems (BioMEMs) targeted to the promotion of bone growth/regeneration by means of coating a biomedical metallic substrate with a functionalized ferroelectric / piezoelectric biocompatible oxide layer or with a functionalized piezoelectric biocompatible / biodegradable polymeric layer and a method to produce said BioMEMs.

This technology relates to BioMEMs comprising a 316L-type stainless steel substrate coated with biocompatible ferroelectric / piezoelectric layers or biocompatible / biodegradable piezoelectric layers and a method in which the 316L-type stainless steel substrates are coated with biocompatible ferroelectric / piezoelectric LiTaO₃ layers or biocompatible / biodegradable piezoelectric polymer layers electrically functionalized via electrical charging by corona poling or UV-light irradiation.

The biocompatible ferroelectric / piezoelectric layers are LiTaO₃ (LTO). In another embodiment the biocompatible / biodegradable piezoelectric layers are poly L-lactic acid (PLLA).

In one embodiment the present technology relates to a method to produce BioMEMs comprising a 316L-type stainless steel substrate coated with functionalized LTO layers.

In another embodiment the present technology relates to a method to produce BioMEMs comprising a 316L-type stainless steel substrate coated with functionalized PLLA layers.

More specifically, the present technology relates to BioMEMs and a method to produce said BioMEMs with biomedical metallic 316L-SST substrates coated with functionalized LTO or PLLA coatings that present a higher surface wettability and energy, a higher rate of calcium phosphate formation and a higher adsorption of BSA proteins.

Furthermore, the present technology relates to a method to produce biomedical metallic 316L-SST substrates coated with functionalized LTO coatings that present no inflammatory reactions for *in vivo* implanted 316L-SST substrates coated with functionalized LTO.

BioMEMs based on 316L-type stainless steel substrates and coated with biocompatible ferroelectric / piezoelectric LTO layers or biocompatible / biodegradable piezoelectric PLLA layers electrically functionalized are suitable to: i) promote bone growth/regeneration; ii) reduce bone implant failure; iii) substitute permanent bone implants; iv) enhance biological *in vivo* or ex *vivo* tissue growth applications; v) improve protein adsorption.

The novelty of this technology lies on the: i) development of a biological platform composed of ferroelectric / piezoelectric LTO or piezoelectric PLLA and 316L-type stainless steel substrates, to be used in the implant technology.

The method includes:
i) preparation of ferroelectric / piezoelectric LTO solutions, or piezoelectric PLLA solutions;
ii) deposition of ferroelectric / piezoelectric LTO layers / coatings or piezoelectric PLLA layers / coatings onto metallic substrates;
   ii.a) repeating the said deposition cycle until the final coating thickness;
   ii.b) crystallizing the said coatings at a temperature commensurate with the crystallization temperature of the deposited layers;
iii) functionalization of ferroelectric / piezoelectric LTO layers / coatings or piezoelectric PLLA layers / coatings by electric charging via corona discharge system;
iv) functionalization of ferroelectric / piezoelectric LTO layers / coatings by photofunctionalization via UV-irradiation.

### Brief description of drawings

The following figures provide preferred embodiments for illustrating the description and should not be seen as limiting the scope of invention. The following figures refer to LTO coatings.

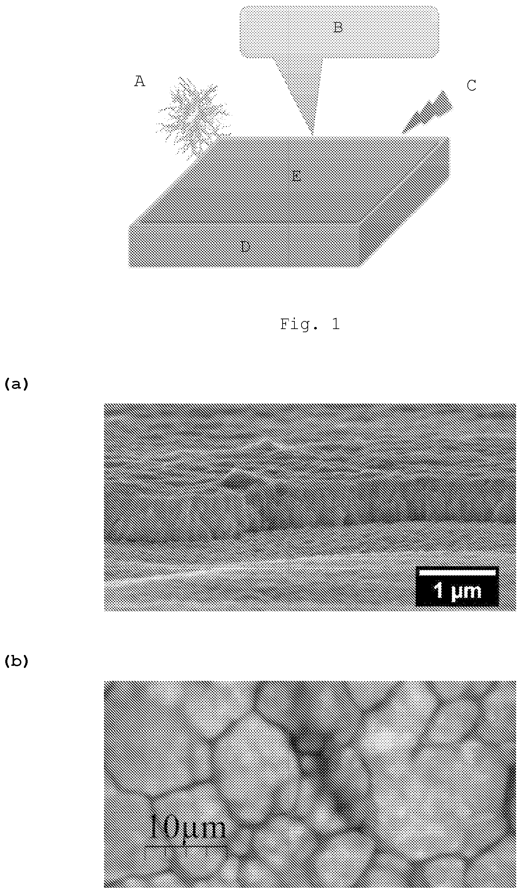

Figure 1: The schematic showing a concept of ferroelectric / piezoelectric LTO coatings onto metallic substrates 316L-SST, either functionalized by electric field (corona poling) - N-LTO/316L-SST, or UV light (photofunctionalization) - UV-LTO/316L-SST. The references are as follows: A - Electrical charging (corona poling) N-LTO/316L-SST; B - Ferroelectric / piezoelectric coating; C - Photofunctionalization (UV-irradiation) UV-LTO/316L-SST; D - 316L-SST; E - LTO/316/L-SST.

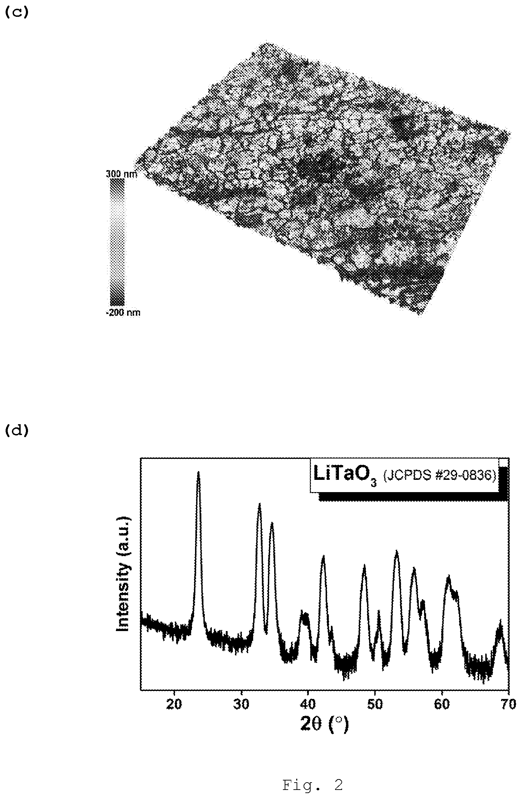

Figure 2: The topological, microstructural and structural characteristics of the as-deposited LTO films on 316L stainless steel (LTO/316L-SST): (a) cross-sectional SEM micrograph with LTO coating thickness between 400 and 600 nm; (b) top view AFM micrograph; (c) the surface topography analysis from the optical profilometry in the form of 3D images; and (d) XRD pattern presenting monophasic LTO structure (JCPDS-PDF #29-0836). Monophasic, well-developed and uniform ferroelectric / piezoelectric LTO layers are coated on 316L-SST metallic substrates.

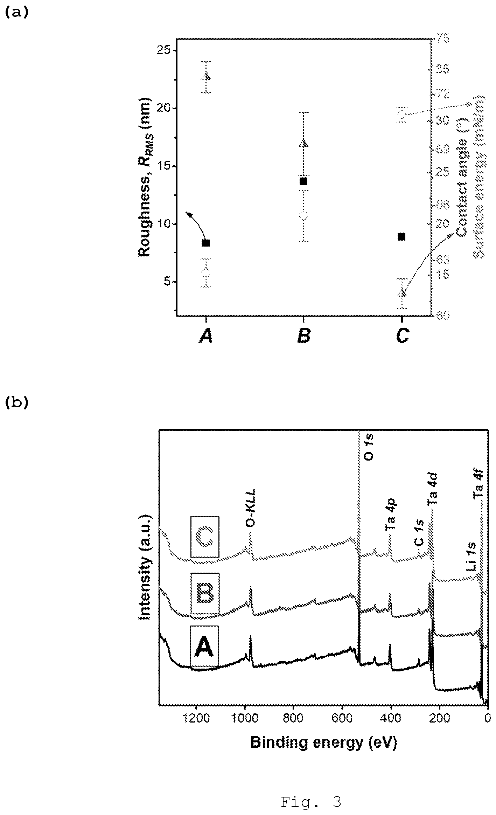

Figure 3: The results of the functionalized ferroelectric / piezoelectric LTO surface analyses: (a) surface roughness, R_{RMS} (left y axis), contact angle and surface energy (right y axis) of as-deposited (A - LTO/316L-SST), electrically treated (B - N-LTO/316L-SST) and photofunctionalized (C - UV-LTO/316L-SST) LTO onto 316L-SST; and (b) general scan of a X-ray photoelectron spectroscopy (XPS) of as-deposited LTO (A) and functionalized LTO (B and C) onto 316L-SST.

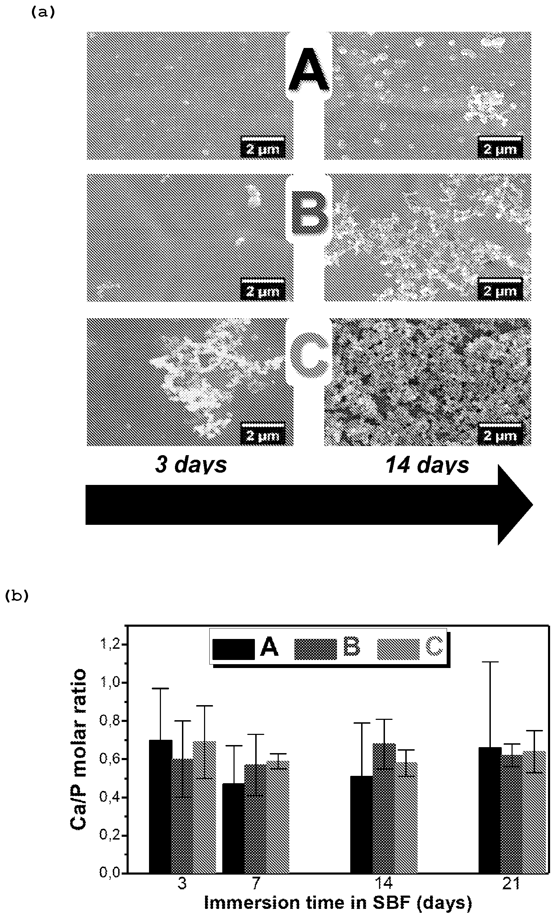

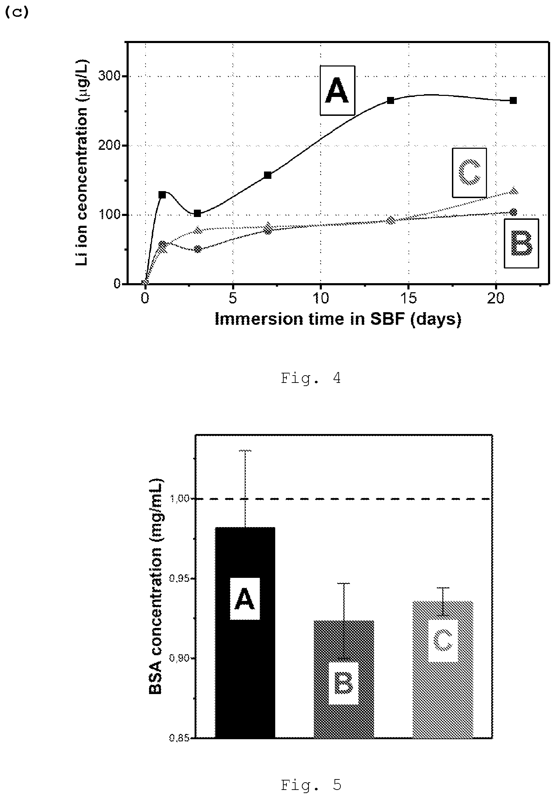

Figure 4: *In vitro* biomineralization results: (a) top view SEM micrographs of as-deposited LTO (LTO/316L-SST - top raw (A), the arrow represents the SBF immersion period) and functionalized LTO (N-LTO/316L-SST - middle raw (B), and UV-LTO/316L-SST - bottom raw (C)) onto 316L-SST incubated in SBF solution for 3 (left column) and 14 (right column) days; (b) Ca/P molar ratio of the precipitates estimated from EDS analysis; and (c) Li ion concentration determined by ICPS analysis versus soaking time in SBF.
Figure 5: *In vitro* BSA protein adsorption results: quantified BSA solution concentration subjected to the assays determined by UV-Vis spectrometry. The highest BSA adsorption is detected on N-LTO/316L-SST (B), while the lowest on LTO/316L-SST (A).

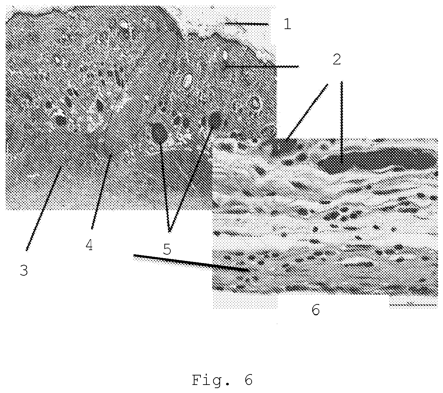

Figure 6: In vivo results for the ferroelectric / piezoelectric coating with no inflammatory reactions for the period of 15 days; skin tissue (1), blood vessels (vascularization) (2), muscle tissue (3), incision area (4), hair follicle (5), 15 days LTO/316L-SST (6).

### Detailed description

The present invention and the scope thereof is defined by the appended claims. The more generic description of the invention is provided for illustrative purposes only. Embodiments not falling under these claims are for reference purposes only.

The present application discloses BioMEMS targeted to the promotion of bone growth by means of coating a biomedical metallic substrate with a functionalized ferroelectric / piezoelectric biocompatible oxide layer or with a functionalized piezoelectric biocompatible and biodegradable polymeric layer. This technology also relates to a method in which biomedical metallic substrates are coated with biocompatible ferroelectric / piezoelectric layers or biocompatible / biodegradable piezoelectric layers electrically functionalized via electrical charging by corona poling or UV-light irradiation.

In one embodiment the BioMEMs comprise a biomedical metallic substrate coated with a functionalized ferroelectric / piezoelectric biocompatible oxide layer or with a functionalized piezoelectric biocompatible / biodegradable polymeric layer.

The biomedical metallic substrate in the BioMEMS is a 316L-SST substrate.

The biocompatible ferroelectric / piezoelectric layers are LTO. In another embodiment the biocompatible / biodegradable piezoelectric polymeric layers are PLLA.

The technology relates to a method to produce BioMEMs comprising a 316L-type stainless steel substrate coated with functionalized LTO layers. In another embodiment the technology relates to a method to produce BioMEMs comprising a 316L-type stainless steel substrate coated with functionalized PLLA layers.

316L-SST substrates coated with functionalized LTO coatings present a higher surface wettability and energy, a higher rate of calcium phosphate formation and a higher adsorption of BSA proteins. No inflammatory reactions were observed for *in vivo* implanted 316L-SST substrates coated with functionalized LTO.

In one embodiment for biocompatible ferroelectric / piezoelectric LTO layers or piezoelectric polymer layers the method comprises the following steps:
i) Preparation of ferroelectric / piezoelectric LTO or piezoelectric PLLA precursor solutions.
   - In one embodiment for LTO solutions: Air-stable LTO precursor solution are prepared by sol-gel method from environmentally friendly components. The air-stable and precipitation-free diol-based precursor solution are prepared using lithium acetate, LiOOCCH₃, and tantalum pentoxide, Ta(OC₂H₅)₅, as reagents and 1,3-propanediol, HO(CH₂)₃OH, as solvent. The metal precursors are separately refluxed under dried nitrogen atmosphere for 8 h in 1,3-propanediol with molar ratio 1:5. Then, the Li(I) and Ta(V) solutions are mixed according to the required stoichiometric molar ratio of the metals, and the final precursor solution is adjusted to a concentration of 0.2 M by adding absolute dry ethanol, HOC₂H₅.
   - In one embodiment for polymer PLLA solutions: PLLA acid precursor solution is prepared by dissolving PLLA pellets in 1,4 dioxane between 70°C and 90°C, more specifically at 80 °C, which is then stabilized at a temperature between 20°C and 30°C.
ii)Deposition of ferroelectric / piezoelectric LTO or piezoelectric polymer layers / coatings onto metallic substrates. Using the previous precursor solutions, the layers / coatings are deposited onto biomedical metallic substrates by spin-coating / dip-coating / spraying, until the final coating thickness is achieved. In between multiple deposition cycles, each of the coatings are dried between 200 and 400°C for 30 s up to 10 min and crystallized between 550 and 650 °C for 1 to 5 min in static O₂ atmosphere by Rapid Thermal Annealing.
iii) Functionalization of ferroelectric / piezoelectric LTO or piezoelectric polymer layers / coatings by electric charging via corona discharge system. In one embodiment the functionalization of ferroelectric / piezoelectric LTO layers hereafter is designated as N-LTO/316L-SST. Corona poling consists in a non-contact poling method in which the air becomes conductive by applying a high electrical field. The procedure was conducted in the vicinity of an electrode with small radius of curvature, sharp tip to generate the discharge made of brass from 100 to 150 mm long and a tip diameter of 0.2 to 0,5 mm, the corona tip, being located at a certain distance, defined by the equipment, from the counter electrode (a grounded conductive plate) that acts as support (being in contact) for the dielectric LTO coatings on 316L-SST. The distance between the tip and the film was set up to 15 mm. The charge flowing from the sharp corona tip accumulates on the dielectric layer due to an ionization effect, and a static electric field over the coating orients the dipoles (within the ferroelectric domains) along the electric field direction. A negative high dc voltage of up to 15 kV was applied for up to 60 min at up to 100 °C and kept constant while cooling down between 15°C and 25°C, more specifically to 20°C (for another time period up to 30 min).
iv)Functionalization of ferroelectric / piezoelectric LTO layers / coatings by photofunctionalization approach via UV-irradiation (UV-LTO/316L-SST in the case of LTO layers with 316L-SST substrate), i.e. photogenerated charge carriers, electrons from the valence band of semiconductors, are activated to the conduction band generating electron-hole pairs that migrate to the surface. In this process the main requirement to generate electron-hole pairs is that the energy of the incident light is larger than the band gap energy of the semiconductors. Thus, in the case of LTO with a band gap of approximately 4.7 eV the wavelength of the UV-light should be lower than 265 nm (UVC range). A lamp emitting light in the UVC range, located at a distance of up to 50 mm to the LTO coatings on 316L-SST and for the period of up to 15 h was used.

In the photofunctionalization step by UV radiation the main requirement to generate electron-hole pairs is that the energy of the incident light is larger than the band gap energy of the semiconductors. Thus, in the case of LTO with a band gap of approximately 4.7 eV the wavelength of the UV-light should be lower than 265 nm (UVC range).

The results obtained clearly demonstrate that functionalization of LTO coatings on 316L-SST by external stimuli, electric field or UV-light, favour the formation of polar groups (carboxyl and carbonyl ones) on the surface of LTO coatings what leads to the increase of: i) surface wettability and energy, ii) rate of calcium phosphate formation, and iii) adsorption of BSA proteins. This enhancement of the bioactivity is independent on the type of functionalization.

XPS results showed that the functionalization treatments by electrical discharge and UV-light both increments the presence of polar groups on the surface and changes the ratio between carbonyl and carboxyl groups. In fact, the proportion of each of these groups depends on the nature of the functionalization. UV-light favors carbonyl groups (C = O), while Corona discharge favors carboxyl ones (-COOH). Consequently, the functionalized surfaces become more hydrophilic and with higher surface energy when compared with the non-functionalized ones. The more hydrophilic surfaces may interact easier with the charged and polar functional groups of proteins and cells; what indeed was observed in the study for BSA proteins.

In one embodiment for *in-vivo* biocompatibility tests the method comprises the norm ISO 10993-1:2009: Biological evaluation of medical devices. LTO/316L-SST functionalised by Corona Charging and UV light were implanted subcutaneously from cranial to caudal aspect of Charles River Laboratories rats.

### Examples

Some examples, for the case biocompatible ferroelectric / piezoelectric LTO layers, are now presented with reference to the figures, which however should not be seen as limiting the scope of technology.

### 1) The concept

Metallic 316L-SST substrates were coated with biocompatible ferroelectric / piezoelectric LTO layers electrically functionalized by corona poling or UV-light irradiation (Figure 1).

### 2) The solutions

The first step for this realization consisted in the preparation of the precursor solutions. An air-stable LTO precursor solution was prepared by sol-gel method using environmentally friendly components.

The air-stable and precipitation-free diol-based precursor solution was prepared using lithium acetate, LiOOCCH₃, and tantalum pentoxide, Ta(OC₂H₅)₅, as reagents and 1,3-propanediol, HO(CH₂)₃OH, as solvent. The metal precursors were separately refluxed under dried nitrogen atmosphere for 8 h in 1,3-propanediol with molar ratio 1:5. Then, the Li(I) and Ta(V) solutions were mixed according to the required stoichiometric molar ratio of the metals, and the final precursor solution was adjusted to a concentration of 0.2 M by adding absolute dry ethanol, HOC₂H₅.

### 3) The coatings

LTO coatings were deposited onto 0.38 mm thick 316L-SST substrates by spin-coating / dip-coating / spraying. Eight deposition cycles with a spinning rate between 1000 and 3000 rpm up to 30 s was carried out until the final film thickness was achieved. The bare metallic substrate was characterized by an average grain size lower than 30 µm. The multiple deposition cycles were carried out until the final coating thickness was achieved. In between cycles, each of the coatings was dried at 250 °C for 1 min and crystallized at 600 °C for 1-5 min in static O₂ atmosphere by Rapid Thermal Annealing. LTO coatings were dense, with a good adhesion to the substrate and with no visible interfacial debonding or surface microcracks. A very uniform coverage of the substrate was obtained with an estimated thickness between 400 and 600 nm and a grain size of LTO of 140 to 160 nm (Figure 2 (a)-(c)); LTO coatings were well-crystallized and chemically monophasic (Figure 2 (d)). The roughness of the coatings deposited on 316L-SST was R_{RMS} 62 nm (root mean square of the roughness).

### 4) The coatings functionalization

For the functionalization with an electric field, corona poling was used. The procedure was conducted in the vicinity of an electrode with small radius of curvature, sharp tip to generate the discharge made of brass from 100 to 150 mm long and a tip diameter of 0.2 to 0,5 mm, the corona tip, being located at a certain distance, defined by the equipment, from the counter electrode (a grounded conductive plate) that acts as support (being in contact) for the dielectric LTO coatings on 316L-SST. A negative high dc voltage of up to 15 kV was applied for up to 60 min at up to 100 °C and kept constant while cooling down between 15°C and 25°C, more specifically to 20°C (for up to 30 min).

For photofunctionalization UV-irradiation was used. Light illumination reactions in semiconductors photogenerated charge carriers, i.e. electrons from the valence band of semiconductors, are activated to the conduction band generating electron-hole pairs that migrate to the surface.

In the case of LTO with a band gap of aproximately 4.7 eV the wavelength of the UV-light should be lower than 265 nm (UVC range). The same UVC light is highly antimicrobial and can be directly applied to acute wound infections to kill pathogens without unacceptable damage to host tissue. UVC is already widely applied for sterilization of inanimate objects.

After the surface functionalization by corona poling and UV irradiation the surface roughness of LTO coatings only slightly changed (<15%) (Figure 3 (a)). However, in terms of functional groups XPS analysis (Figure 3 (b)) revealed that in functionalized LTO either carbonyl or carboxyl functional groups were dominant comparing to as-prepared LTO on 316L-SST.

Figure 3 shows that surface roughness marginally increased after functionalization, with the values within experimental errors. The contact angle values decreased for LTO surface treated coatings, indicating a more hydrophilic surface than the as-prepared one. Surface energy calculated from the contact angle data points to the presence of new functional groups on the surfaces of functionalized films when comparing to as-deposited LTO. It was detected that the carbonyl or carboxyl functional groups are higher in content in functionalized LTO than as-prepared LTO.

### 5) In vitro Biomineralization

After the soaking in simulated body fluid (SBF) a white deposit was observed on all the samples (Figure 4 (a)). The particulate concentration increased with incubation time, being more visible for functionalized coatings where the particulate rate formation was also higher. Vibrational spectroscopies (infrared spectroscopy) confirmed the apatitic nature of the precipitates. By Inductively Coupled Plasma Spectroscopy (ICPS) Li ions concentration detected in SBF solutions reached up to 265 µg/L in the case of as-deposited LTO and being reduced to up to 120 µg/L for functionalized LTO coatings (Figure 4 (c)).

Figure 4 shows that functionalized LTO surfaces promoted the precipitates formation, being it more obvious for UV-irradiated surfaces and longer soaking times. The Ca/P molar ratio of the precipitates is independent on the surface functionalization of LTO and soaking time. The ICPS results indicated that there is Li lixiviation from LTO films to SBF; the biggest Li release occurred within one day (129 µg/L for LTO/316L-SST, 50 µg/L for N-LTO/316L-SST and UV-LTO/316L-SST), reaching 260 µg/L for LTO/316L-SST, and 100 µg/L for N-LTO/316L-SST and UV-LTO/316L-SST (for the 21 days test samples). The levels of Li lixiviation observed did not pose any concern, since the level was quite low, in the range of µg/L. Lithium in doses ≤10 mg/L is currently used for treatment of bipolar disorder patients and other mental problems [27]. For Li concentrations of 10 mg/L of blood, a human being is mildly poisoned, for 15 mg/L they experience confusion and speech impairment, and at 20 mg/L there is a risk of death [28].

### 6) Protein adsorption

The adsorption of proteins is critical within the sequence of biological activities. The effect of coatings functionalization on the adsorption of bovine serum albumin (BSA) revealed clearly a higher protein adsorption at the LTO functionalized surfaces (Figure 5). In addition, the conformation of the adsorbed BSA on various LTO surfaces was different (confirmed by infrared spectroscopy).

Figure 5 shows that the highest BSA adsorption was detected on N-LTO/316L-SST, while the lowest on LTO/316L-SST.

The following amount of BSA proteins was adsorbed: 1.8 ± 4.9 % in the case of LTO/316L-SST, 7.6 ± 2.4 % for N-LTO/316L-SST, and 6.4 ± 0.9 % for UV-LTO/316L-SST. There was a clearly higher protein adsorption at the LTO functionalized surfaces. The difference between both types of functionalized surfaces was not statistically relevant. Protein adsorption was tested with BSA, however it can be considered with any other protein.

UV irradiation noticeably reduces the contact angle of water droplets on LTO coatings by up to 60 % when comparing to non-functionalized surfaces. The surface tension, γSV, of LTO coatings was calculated utilizing the contact angle data and using the equation of state approach, and for LTO/316L-SST was 62.34 ± 0.76 mJ/m2, and increased to 65.45 ± 1.39 mJ/m2 for N-LTO/316L-SST and to 70.90 ± 0.40 mJ/m2 for UV-LTO/316L-SST. The wettability of these functionalized coatings increased as well.

Visual and physical inspection of coated metallic 316L-SST substrates with charged, functionalized via electrical charging or UV-light irradiation, ferroelectric / piezoelectric LTO, revealed excellent quality, a very uniform coverage of the substrate with an estimated thickness between 400 and 600 nm. Coatings were dense, with a good adhesion to the substrate and with no visible interfacial debonding or surface microcracks. The surface topography was not drastically modified by the functionalization treatments. Coating metallic 316L-SST substrates with charged, functionalized via electrical charging or UV-light irradiation, ferroelectric / piezoelectric LTO or piezoelectric PLLA layers, enhanced the formation of calcium phosphates and protein adsorption. The protein conformation is sensitive to the type of charge-functionalization of the ferroelectric / piezoelectric coating and no inflammatory *in vivo* reactions occurred for the period of 15 days.

### 7) In-vivo biocompatibility

In-vivo biocompatibility tests were done according to the ISO 10993-1:2009 standard: Biological evaluation of medical devices. LTO/316L-SST functionalized by corona charging and UV light were implanted subcutaneously from cranial to caudal aspect of Charles River Laboratories rats. Implants systematic and periodic inspection revealed: i) no inflammatory signs ii) good cicatrisation of the tissues in incision area: implant area was confined in shape, borders showed good smooth aspect and adjacent tissues evidenced normal tissue growth and positive vascularization; iii) low inflammation response (according with the ISO standards for medical implants); iv) presence of very low number (rare) of polymorphonuclear cells, giant cells, plasma cells and/or degradation of materials; v) in general from score analysis the test samples were considered non-irritant to slight irritant (maximum score = 3.8 at 7 days of implantation).

### References

1. Amini, A. R.; Laurencin, C. T.; Nukavarapu, S. P., Bone Tissue Engineering: Recent Advances and Challenges. Critical Reviews in Biomedical Engineering 2012, 40 (5), 363-408.
2. Mouriño, V.; Boccaccini, A. R., Bone tissue engineering therapeutics: controlled drug delivery in three-dimensional scaffolds. Journal of the Royal Society Interface 2010, 7 (43), 209-227.
3. Bohner, M., Resorbable Biomaterials as Bone Graft Substitutes. Materials Today 2010, 13 (1-2), 24-30.
4. Chen, Q.; Thouas, G. A., Metallic Implant Biomaterials. Materials Science and Engineering: R: Reports 2015, 87, 1-57.
5. Wang, W.; Ouyang, Y.; Poh, C. K., Orthopaedic implant technology: Biomaterials from past to future. Annals of the Academy of Medicine Singapore 2011, 40 (5), 237-243.
6. Goodman, S. B.; Yao, Z.; Keeney, M.; Yang, F., The future of biologic coatings for orthopaedic implants. Biomaterials 2013, 34 (13), 3174-3183.
7. Shah, N. J.; Hong, J.; Hyder, M. N.; Hammond, P. T., Osteophilic multilayer coatings for accelerated bone tissue growth. Advanced Materials 2012, 24 (11), 1445-1450.
8. Xiao M.; Chen Y. M.; Biao M. N.; Zhang X. D.; Yang B. C., Bio-functionalization of biomedical metals, Materials Science and Engineering C, 70, 1057-1070, 2017*.)*
9. Active bio piezoelectric ceramic coating layer and method of preparing said coating layer on titanium base body surface, Patent CN 1785439 A, Publication date 8 Oct 2008.
10.Catauro M.; Bollino F.; Giovanardi R.; Veronesi P., Modification of Ti6A14V implant surfaces by biocompatible TiO2/PCL hybrid layers prepared via sol-gel dip coating: structural characterization, mechanical and corrosion behavior, , Materials Science & Engineering C-Materials For Biological Applications, 74, 501-507, 2017.
11. Tasnim N. ; Kumar A.; Joddar B., Attenuation of the in vitro neurotoxicity of 316L SS by graphene oxide surface ncoating, Materials Science & Engineering C-Materials For Biological Applications, 73, 788-797, 2017*.*
12.Zhao, M., Electrical Fields in Wound Healing - An Overriding Signal that Directs Cell Migration. Seminars in Cell & Developmental Biology 2009, 20 (6), 674-682.
13.Yao, L.; McCaig, C. D.; Zhao, M., Electrical Signals Polarize Neuronal Organelles, Direct Neuron Migration, and Orient Cell Division. Hippocampus 2009, 19 (9), 855-868.
14.Levin, M., Bioelectric mechanisms in regeneration: Unique aspects and future perspectives. Seminars in Cell & Developmental Biology 2009, 20 (5), 543-556.
15.Radisic, M.; Park, H.; Shing, H.; Consi, T.; Schoen, F. J.; Langer, R.; Freed, L. E.; Vunjak-Novakovic, G., Functional assembly of engineered myocardium by electrical stimulation of cardiac myocytes cultured on scaffolds. Proceedings of the National Academy of Sciences 2004, 101 (52), 18129-18134.
16.Aaron, R. K.; Boyan, B. D.; Ciombor, D. M.; Schwartz, Z.; Simon, B. J., Stimulation of growth factor synthesis by electric and electromagnetic fields. In Clinical Orthopaedics and Related Research, 2004; pp 30-37.
17.Zhao, M.; Song, B.; Pu, J.; Wada, T.; Reid, B.; Tai, G.; Wang, F.; Guo, A.; Walczysko, P.; Gu, Y.; Sasaki, T.; Suzuki, A.; Forrester, J. V.; Bourne, H. R.; Devreotes, P. N.; McCaig, C. D.; Penninger, J. M., Electrical signals control wound healing through phosphatidylinositol-3-OH Kinase-g and PTEN. Nature 2006, 442 (7101), 457-460.
18.Baxter, F. R.; Bowen, C. R.; Turner, I. G.; Dent, A. C. E., Electrically Active Bioceramics: A Review of Interfacial Responses. Ann Biomed Eng 2010, 38 (6), 2079-2092.
19.Barroca, N.; Vilarinho, P. M.; Fernandes, M. H. V.; Sharma, P.; Gruverman, A., Stability of electrically induced-polarization in poly (L-lactic) acid for bone regeneration. Applied Physics Letters 2012, 101 (2), 023701.
20.Carville, N. C.; Collins, L.; Manzo, M.; Gallo, K.; Lukasz, B. I.; McKayed, K. K.; Simpson, J. C.; Rodriguez, B. J., Biocompatibility of ferroelectric lithium niobate and the influence of polarization charge on osteoblast proliferation and function. Journal of Biomedical Materials Research Part A 2014, 103 (8), 2540-2548.
21.Dubey, A. K.; Basu, B., Pulsed Electrical Stimulation and Surface Charge Induced Cell Growth on Multistage Spark Plasma Sintered Hydroxyapatite-Barium Titanate Piezobiocomposite. Journal of the American Ceramic Society 2014, 97 (2), 481-489.
22.Nilsson, K.; Lidman, J.; Ljungstrom, K.; Kjellman, C. Biocompatible material for implants. 2003.
23.Wang, Q.; Yang, J.; Zhang, W.; Khoie, R.; Li, Y.-m.; Zhu, J.-g.; Chen, Z.-q., Manufacture and Cytotoxicity of a Lead[hyphen]free Piezoelectric Ceramic as a Bone Substitute[mdash]Consolidation of Porous Lithium Sodium Potassium Niobate by Cold Isostatic Pressing. Int J Oral Sci 2009, 1 (2), 99-104.
24.Christophis, C.; Cavalcanti-Adam, E.; Hanke, M.; Kitamura, K.; Gruverman, A.; Grunze, M.; Dowben, P.; Rosenhahn, A., Adherent cells avoid polarization gradients on periodically poled LiTaO3 ferroelectrics. Biointerphases 2013, 8 (1), 1-9.
25.Marchesano, V.; Gennari, O.; Mecozzi, L.; Grilli, S.; Ferraro, P., Effects of Lithium Niobate Polarization on Cell Adhesion and Morphology. ACS Applied Materials & Interfaces 2015, 7 (32), 18113-18119.
26.Vilarinho, P. M.; Barroca, N.; Zlotnik, S.; Félix, P.; Fernandes, M. H., Are lithium niobate (LiNbO3) and lithium tantalate (LiTaO3) ferroelectrics bioactive? Materials Science and Engineering: C 2014, 39, 395-402.
27.Viguera, A.; Cohen, L.; Baldessarini, R.; Nonacs, R. Managing bipolar disorder during pregnancy: weighting the risks and benefits. Canadian Journal of Psychiatry 2002, 47, 426-36.
28.Miyazaki, T.; Kim, H.M.; Kokubo, T.; Kato H. Induction and acceleration of bonelike apatite formation on tantalum oxide gel in simulated body fluid. Journal of Sol-Gel Science and Technology 2001, 21, 1-2, 83-88.

## Claims

1. BioMicroElectroMechanical System targeted to promote bone regeneration comprising:
- a 316L-type stainless steel substrate coated with:
- ferroelectric / piezoelectric LiTaO₃ layers;
- or biocompatible / biodegradable piezoelectric polymer layers;
wherein the layers are electrically functionalized and present high surface wettability and energy, high rate of calcium phosphate formation, high adsorption of proteins and no inflammatory reactions in vivo.

2. BioMicroElectroMechanical System according to any of the previous claims, wherein the biocompatible / biodegradable polymer layers are poly L-lactic acid.

3. Method for obtaining a BioMicroElectroMechanical System as described in any of the previous claims comprising the following steps:
- preparation of ferroelectric / piezoelectric LTO precursor solutions, or biocompatible / biodegradable piezoelectric polymer precursor solutions;
- deposition of ferroelectric / piezoelectric LTO layers / coatings or biocompatible / biodegradable piezoelectric polymer layers / coatings onto 316L-type stainless steel substrates by spin-coating / dip-coating / spraying;
- repeating the said deposition cycle until the final coating thickness;
- crystallizing the said coatings at a temperature commensurate with the crystallization temperature of the deposited layers;
- functionalization of ferroelectric / piezoelectric LTO layers / coatings or biocompatible / biodegradable piezoelectric polymer layers / coatings via electric field or photofunctionalization via UV-irradiation;
wherein the coatings functionalization with an electric field is done by corona poling conducted in the vicinity of an electrode with small radius of curvature, the corona tip, being located at a distance from the counter electrode which a grounded conductive plate, defined by the equipment, that acts as support for the coatings on the 316L-type stainless steel substrate;
and wherein the coatings photofunctionalization occurs with a wavelength of the UV-light lower than 265 nm.

4. Method according to claim 3, wherein the formation of a LTO precursor solution comprises the following steps:
- preparation of an air-stable precursor solution by sol-gel method from environmentally friendly components;
- the metal precursors are mixed according to the required stoichiometric molar ratio of the metals;
- the final precursor is adjusted to a certain concentration.

5. Method according to claim 3, wherein the preparation of a precursor polymer solution is made by dissolving poly L-lactic acid pellets in 1,4 dioxane at between 70°C and 90°C, which is then stabilized at a temperature between 20°C and 30°C.

6. Method according to any of the claims 3 to 5, wherein the deposition of the coatings comprises the following steps:
- using the previously prepared precursor solutions;
- multiple deposition cycles of the precursor solutions until the final coating thickness is achieved;
- in between cycles, each of the coatings is dried between 200 and 400°C for 30 s to 10 min and crystallized between 550 and 650 °C for 1 to 5 min in static O₂ atmosphere by Rapid Thermal Annealing.

7. Method according to claim 3, wherein a negative high dc voltage of up to 15 kV is applied for up to 60 min at up to 100 °C and kept constant while cooling down between 15°C and 25°C, more specifically to 20°C.

## Patentansprüche

1. Biomikroelektromechanisches System zur Förderung der Knochenregeneration, bestehend aus:
- Einem 316L Edelstahl-Substrat, beschichtet mit:
- Ferroelektrischen / piezoelektrischen LiTaO₃ Schichten;
- oder biokompatiblen / biologisch abbaubaren piezoelektrischen Polymerschichten;
worin die Schichten elektrisch funktionalisiert werden und eine hohe Oberflächenbenetzbarkeit und Energie, eine hohe Kalziumphosphat Bildungsrate, hohe Adsorption von Proteinen und keine Entzündungsreaktionen in vivo aufweisen.

2. Biomikroelektromechanisches System gemäß einem der vorhergehenden Ansprüche, worin die biokompatiblen / biologisch abbaubaren Polymerschichten Poly-L-Milchsäure sind.

3. Methode zur Erlangung eines Biomikroelektromechanischen Systems, wie in einem der vorherigen Ansprüche beschrieben, das die folgenden Schritte umfasst:
- Vorbereitung von ferroelektrischen/piezoelektrischen LTO-Vorstufenlösungen oder biokompatiblen / biologisch abbaubaren piezoelektrischen Polymer-Vorstufenlösungen;
- Ablagerung von ferroelektrischen / piezoelektrischen LTO-Schichten / Beschichtungen oder biokompatiblen / biologisch abbaubaren piezoelektrischen Polymerschichten / Beschichtungen auf den 316L Edelstahlsubstraten durch Spin-Coating / Tauchbeschichtung / Sprühen;
- Wiederholung des genannten Ablagerungszyklus bis zum Erreichen der endgültigen Schichtdicke;
- Kristallisieren der genannten Beschichtungen bei einer Temperatur, die der Kristallisationstemperatur der abgelagerten Schichten entspricht;
- Funktionalisierung der ferroelektrischen / piezoelektrischen LTO-Schichten / Beschichtungen oder der biokompatiblen / biologisch abbaubaren piezoelektrischen Polymerschichten / Beschichtungen über ein elektrisches Feld oder durch Photofunktionalisierung über UV-Bestrahlung;
worin die Funktionalisierung der Beschichtungen mit einem elektrischen Feld durch Koronapolung erfolgt, die in der Nähe einer Elektrode mit kleinem Krümmungsradius durchgeführt wird, wobei sich die Koronaspitze in einem Abstand von der Gegenelektrode befindet, die eine geerdete, durch die Ausrüstung definierte leitfähige Platte ist, welche als Halterung für die Beschichtungen auf dem 316L Edelstahl-Substrat dient;
und worin die Photofunktionalisierung der Beschichtungen mit einer Wellenlänge des UV-Lichts unter 265 nm erfolgt.

4. Methode gemäß Anspruch 3, worin die Bildung einer LTO-Vorstufenlösung die folgenden Schritte umfasst:
- Vorbereitung einer luftbeständigen Vorstufenlösung mittels einer Sol-Gel-Methode aus umweltfreundlichen Komponenten;
- die Metallvorstufen werden gemäß dem erforderlichen stöchiometrischen Molverhältnis der Metalle gemischt;
- Der letzte Vorstufe wird auf eine bestimmte Konzentration eingestellt.

5. Methode gemäß Anspruch 3, worin die Vorbereitung einer Vorstufen-Polymerlösung durch das Auflösen von Poly-L-Milchsäurepellets in 1,4 Dioxan bei 70 bis 90 Grad Celsius erfolgt, die danach bei einer Temperatur zwischen 20 und 30 Grad Celsius stabilisiert wird.

6. Methode gemäß einem der Ansprüche 3 bis 5, worin die Ablagerung der Beschichtungen die folgenden Schritte umfasst:
- Verwendung der zuvor vorbereiteten Vorstufenlösungen;
- mehrere Ablagerungszyklen der Vorstufenlösungen bis die endgültige Schichtdicke erreicht ist;
- zwischen den Zyklen wird jede der Beschichtungen bei Temperaturen zwischen 200 und 400 Grad Celsius für 30 s bis 10 min. getrocknet und zwischen 550 und 650 Grad Celsius für 1 bis 5 min. in statischer O₂-Atmosphäre durch schnelles thermisches Glühen kristallisiert.

7. Methode gemäß Anspruch 3, worin eine negative Hochspannung von bis zu 15 kV für bis zu 60 min. bei bis 100 Grad Celsius angelegt wird, die während des Abkühlens auf 15 bis 25 Grad Celsius, genauer gesagt auf 20 Grad Celsius konstant gehalten wird.

## Revendications

1. Bio-microsystème électromécanique destiné à favoriser une régénération osseuse comprenant :
- un substrat en acier inoxydable de type 316L revêtu de :
- couches ferroélectriques / piézoélectriques de LiTaO₃ ;
- ou de couches polymères piézoélectriques biocompatibles / biodégradables ;
dans lequel les couches sont fonctionnalisées électriquement et présentent une mouillabilité et une énergie de surface élevées, un taux élevé de formation de phosphate de calcium, une adsorption élevée de protéines et pas de réactions inflammatoires in vivo.

2. Bio-microsystème électromécanique selon l'une quelconque des revendications précédentes, dans lequel les couches polymères biocompatibles / biodégradables sont en acide poly L-lactique.

3. Procédé d'obtention d'un Bio-microsystème électromécanique tel que décrit selon l'une quelconque des revendications précédentes comprenant les étapes suivantes :
- préparation de solutions de précurseurs de LTO ferroélectriques / piézoélectriques, ou de solutions de précurseurs de polymères piézoélectriques biocompatibles / biodégradables ;
- dépôt de couches / revêtements ferroélectriques / revêtements ferroélectriques / piézoélectriques de LTO ou de couches / revêtements polymères piézoélectriques biocompatibles / biodégradables sur des substrats en acier inoxydable de type 316L par revêtement par centrifugation / revêtement par immersion / pulvérisation ;
- répétition dudit cycle de dépôt jusqu'à l'épaisseur de revêtement finale ;
- cristallisation desdits revêtements à une température proportionnelle à la température de cristallisation des couches déposées ;
- fonctionnalisation des couches / revêtements ferroélectriques / piézoélectriques de LTO ou de couches / revêtements polymères piézoélectriques biocompatibles / biodégradables par champ électrique ou photofonctionnalisation par irradiation UV ;
dans laquelle la fonctionnalisation des revêtements avec un champ électrique est faite par polarisation corona menée dans le voisinage d'une électrode à faible rayon de courbure, la pointe corona étant située à une certaine distance de la contre-électrode qui est une plaque conductrice mise à la masse, définie par l'équipement, qui agit en tant que support pour les revêtements sur le substrat en acier inoxydable de type 316L ;
et dans laquelle la photofonctionnalisation des revêtements se produit avec une longueur d'onde de la lumière UV inférieure à 265 nm.

4. Procédé selon la revendication 3, dans lequel la formation d'une solution de précurseur de LTO comprend les étapes suivantes :
- préparation d'une solution de précurseur stable à l'air par procédé sol-gel à partir de composants respectueux de l'environnement ;
- les précurseurs métalliques sont mélangés selon le rapport molaire stœchiométrique requis des métaux ;
- le précurseur final est ajusté à une certaine concentration.

5. Procédé selon la revendication 3, dans lequel la préparation d'une solution polymère de précurseur est faite par la dissolution de granulés d'acide poly L-lactiques dans du 1,4 dioxane entre 70 °C et 90 °C, qui est ensuite stabilisée à une température entre 20 °C et 30 °C.

6. Procédé selon l'une quelconque des revendications 3 à 5, dans lequel le dépôt des revêtements comprend les étapes suivantes :
- à l'aide des solutions de précurseur préparées préalablement ;
- plusieurs cycles de dépôts de solutions de précurseur jusqu'à ce que l'épaisseur de revêtement finale soit obtenue ;
- entre les cycles, chacun parmi les revêtements est séché entre 200 et 400 °C pendant 30 s à 10 min et cristallisé entre 550 et 650 °C pendant 1 à 5 min en atmosphère O₂ statique par Recuit Thermique Rapide.

7. Procédé selon la revendication 3, dans lequel une haute tension cc négative de jusqu'à 15 kV est appliquée pendant jusqu'à 60 min à jusqu'à 100 °C et maintenue constante durant le refroidissement entre 15 °C et 25 °C, plus spécifiquement à 20 °C.
